Europäisches Patentamt

European Patent Office

Office européen des brevets

(11) Veröffentlichungsnummer: **0 146 646 B1**

(12) **EUROPÄISCHE PATENTSCHRIFT**

(45) Veröffentlichungstag der Patentschrift:
27.01.88

(51) Int. Cl.⁴: **B 01 D 53/04, C 07 C 7/12, C 01 B 31/20, C 01 B 3/56**

(21) Anmeldenummer: 83112613.1

(22) Anmeldetag: 15.12.83

(54) Verfahren zur Abtrennung und Gewinnung von relativ stark an Adsorptionsmitteln adsorbierbaren Gasen aus ansonsten im wesentlichen nur schwächer adsorbierbare Gase enthaltenden Gasgemischen.

(43) Veröffentlichungstag der Anmeldung:
03.07.85 Patentblatt 85/27

(45) Bekanntmachung des Hinweises auf die Patenterteilung:
27.01.88 Patentblatt 88/4

(84) Benannte Vertragsstaaten:
BE DE FR GB IT NL

(56) Entgegenhaltungen:
DE - A - 1 444 447
DE - A - 2 038 261
DE - A - 2 624 346
GB - A - 2 058 826
US - A - 3 751 878
US - A - 4 077 779
US - A - 4 404 004

(73) Patentinhaber: Bergwerksverband GmbH,
Franz-Fischer-Weg 61, D-4300 Essen 13 (DE)

(72) Erfinder: Richter, Ekkehard, Dr.,
Schmachtenbergstrasse 89, D-4300 Essen 18 (DE)
Erfinder: Körbächer, Werner, Striepensweg 4,
D-4330 Mülheim/Ruhr (DE)
Erfinder: Knoblauch, Karl, Dr., Semperstrasse 55,
D-4300 Essen 1 (DE)
Erfinder: Harder, Burkhard, Dr., Am Barchembach 12,
D-4200 Oberhausen (DE)
Erfinder: Giessler, Klaus, Weindorfstrasse 21,
D-4650 Gelsenkirchen (DE)
Erfinder: Kronauer, Peter, Adelgundenweg 65,
D-4300 Essen 1 (DE)
Erfinder: Tarnow, Ferdinand, Im Ährenfeld 41,
D-4100 Duisburg 25 (DE)

**Beschreibung**

Die Erfindung betrifft ein Verfahren gemäss dem Oberbegriff von Patentanspruch 1.

Man kann Kohlendioxid, Methan und andere, relativ stark an Adsorptionsmitteln adsorbierbare Gase (Produktgaskomponenten) aus solche enthaltenden Gasgemischen abtrennen, indem man die Produktgaskomponenten an, insbesondere kohlenstoffhaltigen, Adsorptionsmittel adsorbiert. Bisher gelang es jedoch nicht, diese adsorbierten Produktgaskomponenten in reiner Form, beispielsweise mit Konzentrationen von über 99,5 Vol.-%, bei der Desorption mit zufriedenstellender Produktgasausbeute zurückzugewinnen. Bei einigen technischen Anwendungsfällen fallen solche Produktgaskomponenten in Mengenanteilen von etwa 15 bis 50 Vol.-% an, so dass ihre Gewinnung durchaus interessant wäre, so z.B.:

- Methan aus Grubengas
- Acethylen aus einem Acethylen/Wasserstoffkreislaufgas
- Kohlenmonoxid aus einem Reformergas
- Kohlendioxid aus Konvertergas oder Rauchgas.

Die Schwierigkeit der Abtrennung von solchen Produktgaskomponenten aus Gasgemischen durch Adsorption liegt bisher darin, dass bei der Desorption eines beladenen Adsorbers auch die gleichzeitig adsorbierten, anderen Gaskomponenten des Ausgangsgasgemisches freigesetzt werden. Auf diese Weise sind in den desorbierten Gasen (Produktgas) auch die im Ausgangsgasgemisch enthaltenen Komponenten mehr oder weniger als Verunreinigungen enthalten.

Aus GB-A-2 058 826 ist ein Verfahren zur Gewinnung von $CH_4$ mittels Druckwechseladsorptionstechnik bekannt, bei dem unter Anwendung der Grundschritte Adsorption, Spülung mit Produktgaskomponenten, Evakuieren und erneuter Druckaufbau Methan in einer Konzentration von 90 bis 95% gewonnen werden kann. Dieses Gas dient als Brennstoff, so dass eine höhere Konzentration offenbar nicht angestrebt wird und auch nicht erzielbar ist. In dem Adsorptionszyklus ist ausserdem ein Druckabsenkungsschritt im Gleichstrom zwischen der Adsorptions- und Desorptionsphase nicht vorgesehen.

In der US-A-3 751 878 ist ein Druckwechseladsorptionsverfahren beschrieben, bei dem ein Ausgangsgas, das $CH_4$ und $CO_2$ enthält, die beide stark adsorbierbar sind, getrennt werden. Das Hauptproduktgas ist $CH_4$, während das $CO_2$ teils verworfen wird, teils als Nebenproduktgas mitgewonnen wird. Obwohl ein Druckentspannungsschritt in dem Adsorptionszyklus ausgewiesen ist, lassen sich nach diesem Verfahren offenbar keine sehr hohen $CH_4$-Konzentrationen bzw. $CO_2$-Konzentrationen erzielen. Die Produktgasausbeuten werden nicht angegeben. Ein Evakuierungsschritt ist in dem Zyklus nicht enthalten.

Weiterhin sind aus DE-A-1 444 447, US-A-4 404 004 und US-A-4 077 779 Druckerniedrigungsstufen als Schrittfolge von Druckwechselprozessen zur Gastrennung bekannt, bei denen diese Teilgasmengen im Druckwechselprozess wiederverwendbar sind. Bei keinem dieser Verfahren gelingt es jedoch durch die jeweils angewendeten Schrittkombinationen Produktgaskonzentrationen über 99% bei gleichzeitig hoher Ausbeute zu erzielen, wenn das Produktgas ein stark adsorbierbares Gas ist und aus Gasgemischen gewonnen werden soll, die ansonsten nur schwach adsorbierbare Gase enthalten.

Der Erfindung liegt die Aufgabe zugrunde, Gasgemische, welche die Produktgaskomponente von unter 15 Vol.-% bis über 50 Vol.-% enthalten, selektiv an Adsorptionsmitteln so zu trennen, dass das Produktgas in einer möglichst hohen Reinheit von z.B. über 99,5 Vol.-% und zugleich in hoher Ausbeute erhalten wird.

Diese Aufgabe wird hinsichtlich eines Verfahrens durch die kennzeichnenden Merkmale von Patentanspruch 1 gelöst. Weiterbildungen werden in den Unteransprüchen aufgeführt.

Die Erfindung basiert auf dem Grundgedanken, bei einem Druckwechseladsorptions-/-desorptionsprozess zur Gewinnung relativ stark adsorbierbarer Produktgaskomponenten, bei dem zwischen der Adsorptions- und Desorptionsphase eine Spülphase (Adsorptionsspülung) eingefügt sein kann, eine Ausbeutesteigerung an Produktgaskomponenten dadurch zu erzielen, dass zwischen dem Ende der Adsorptionsphase und vor dem Beginn der Produktionsphase (Produktgasgewinnung durch Druckerniedrigung) eine besondere Druckabsenkungsphase eingefügt wird, während der der Druck im Adsorber durch Abströmen einer Teilgasmenge im Gleichstrom zur Strömungsrichtung der vorangehenden Arbeitsphase abgesenkt wird. Hierdurch verdrängen Produktgaskomponenten aus dem abströmenden Gas im Endbereich des Adsorbers einen guten Teil der dort noch vorhandenen Abgaskomponenten auf Adsorptionsplätzen des Adsorptionsmittels und im Zwischenlückenvolumen der Adsorptionsmittelschicht. Dieser besondere Druckabsenkungsschritt kann vor oder nach der Spülphase mit Produktgaskomponenten erfolgen. Bevorzugt erfolgt diese Spülphase des Adsorbers im Gleichstrom zur Adsorption und die Adsorptionsphase wird vor Beginn des Durchbruches der Produktgaskomponente(n) durch die Adsorptionsmittelschicht beendet, weil diese Massnahmen in Kombination mit dem besonderen Druckabsenkungsschritt die günstigste Ausbeutesteigerung und beste Produktgasanreicherung bewirken. Nach der Erfindung ist es daher möglich, die Adsorption des Rohgases auf einem deutlich oberhalb von Atmosphärendruck liegenden Gasdruck, insbesondere bei dem Druck durchzuführen, bei dem das Rohgas z.B. aus einem chemischen Prozess anfällt. Diesem Adsorptionsschritt auf erhöhtem Druck kann eine Voradsorptionsstufe auf dem selben Druck vorgeschaltet sein, in der bevorzugt Wasserdampf aus dem Rohgas entfernt wird (Trocknungsstufe). Nach dem Ende der Adsorption erfolgt dann eine Druckabsenkung auf etwa Atmosphärendruck und bei diesem die Spülphase mit einem Gas von etwa Produktgasqualität. Diese Adsorption auf erhöhtem Druck führt zu kleineren Adsorbervolumina und gestattet es, den Druckaufbau eines parallel arbeitenden Adsorbers auf Drücke oberhalb Atmosphärendruck z.T. mit der bei der Druckabsenkung eines anderen Adsorbers zwischen Adssorptionsphase und Spülphase anfallenden Teilgasmenge durchzuführen. Der besondere Druckabsenkungsschritt kann allerdings auch bei einer auf etwa Atmosphärendruck betriebenen Adsorptionsphase in ein Vakuum hinein erfolgen, wobei die nachfolgende Adsorptionsspülung bevorzugt wieder bei etwa Atmosphärendruck erfolgt. Schliesslich kann der besondere Druckabsenkungsschritt auch nach der Adsorptionsspülung erfolgen, wobei entweder ein Druckausgleich mit einem evakuierten Adsorber oder eine besondere Vakuumpumpe zum Einsatz kommen, so dass die Produktgasgewinnung nachfolgend auf einem durch weitere Druckabsenkung noch niedrigeren Druckniveau erfolgt.

Als Produktgaskomponenten werden erfindungsgemäss diejenigen Gaskomponenten des Ausgangsgemisches verstanden, die relativ am stärksten und deshalb im Eingangsbereich der Adsorptionsmittelschicht adsorbieren und die schwächer adsorbierbaren Gaskomponenten beim Fortschreiten der Adsorptionsfront von ihren Adsorptionsplätzen verdrängen; entsprechend umgekehrtes gilt für die schwächer adsorbierbaren Gaskomponenten, die der Einfachheit halber als Abgaskomponenten bezeichnet werden. Dementsprechend beinhaltet das Produktgas einen höheren Volumenanteil an Produktgaskomponenten als das Ausgangsgasgemisch, während das Abgas entsprechend stark an Produktgaskomponenten abgereichert ist.

Der Spülschritt (Spülphase) wird nun mit einem Gasgemisch aus dem Druckwechselprozess durchgeführt, welches an Produktgaskomponenten angereichert ist; hierbei kann das Spülgas im Laufe der Spülphase konzentrationsmässig an der Produktgaskomponente allmählich oder stufenweise zunehmen, in jedem Fall ist aber zum Ende der Spülphase mit einem Gas von etwa Produktgasqualität zu spülen. Das beste Ergebnis wird erzielt, wenn die gesamte Spülung mit einem Gasgemisch von Produktgasqualität erfolgt. Dieser Spülschritt, der eigentlich mehr ein zweiter Adsorptionsschritt ist, der in der Regel auf Atmosphärendruck stattfindet, führt also dazu, dass die noch nicht von den Produktgaskomponenten besetzten Adsorptionsplätze unter Verdrängung der dort adsorbierten Abgaskomponenten mit Produktgaskomponenten aufgefüllt werden. Auf diese Weise ist am Ende der Spülphase das gesamte Adsorptionsmittel mit Produktgaskomponenten belegt, so dass bei der darauffolgenden Druckerniedrigung in der Desorptionsphase nur noch unwesentlich verunreinigtes Produktsgas abströmt. Für eine hohe Ausbeute an der Produktgaskomponente versteht es sich, dass die eigentliche Adsorption dann beendet wird, wenn die Produktgaskomponente kurz vor dem Durchbruch steht oder gerade durchgebrochen ist, und es versteht sich weiterhin, dass die «Spülung» höchstens so lange betrieben wird, bis der Durchbruch von zunächst nur einigen Prozent an Produktgaskomponente annähernd vervollständigt ist. d.h. annähernd 100% Produktgas aus der Adsorptionsmittelschicht abströmen. Das bei der «Spülung» abströmende Gasgemisch kann nach einer längeren Spüldauer eine höhere Produktgaskonzentration als das Ausgangsgasgemisch aufweisen und wird in diesem Falle bevorzugt dem Druckwechselprozess wieder zugeführt, und zwar für die Adsorptionsphase oder die Spülphase; zumindest das gegen Ende der Spülphase abströmende Gasgemisch eignet sich jedoch am besten zum Einsatz für die Adsorptions- bzw. Spülphase in einem parallelen Adsorber, weil dann die Gaskonzentration (Konzentration im abströmenden Gasgemisch) schnell bis auf Produktgasqualität steigt und so den Anforderungen des «Spülschrittes» in besonderem Masse genügt.

Es versteht sich, dass beim Anfahren einer Anlage nach der Erfindung eine gewisse Einlaufzeit erforderlich ist, in der sich die einzelnen Mengenströme und Gaskonzentrationen noch verschieben; entscheidend für die Erfindung ist der nach einer gewissen Zahl von Zyklen erreichte Gleichgewichtszustand.

Besonders vorteilhaft ist eine schnelle und hohe Aufnahmefähigkeit des Adsorptionsmittels für die Produktgaskomponente(n) bei der Adsorptionsphase sowie eine schnelle und möglichst vollständige Desorption der Produktgaskomponente(n) bei der Desorption. Dies wird erfindungsgemäss dadurch erreicht, dass der Druckwechselprozess im wesentlichen adiabatisch geführt wird.

Mit dem Verfahren der Erfindung lassen sich alle relativ stark adsorbierbaren Gase, insbesondere Methan und Kohlendioxid, enthaltende Gasgemische, beispielsweise solche mit Sauerstoff, Stickstoff, Kohlenmonoxid, Wasserstoff, Helium, Argon oder anderen, an dem, vorzugsweise kohlenstoffhaltigen, Adsorptionsmittel schwach adsorbierbaren Gasen, zu einem Produktgas hoher Reinheit und Ausbeute aufarbeiten. Überraschenderweise ist es auch unschädlich, wenn im Ausgangsgemisch auch Spuren, z.B. unter 1 Vol.-%, von noch stärker adsorbierbaren Gaskomponenten als der (den) Produktgaskomponente(n) — das Produktgas kann auch ein besonders interessierendes Gasgemisch aus mehreren etwa gleich stark adsorbierbaren Gaskomponenten sein — vorhanden sind, weil diese in jedem Falle adsorbiert, meistens aber durch Druckerniedrigung allein nicht desorbiert werden, so dass sie das Desorptionsgas (Produktgas) nicht verunreinigen, sondern z.B. in einem sich an die Desorptionsphase anschliessenden Spülschritt entfernt werden können; man kann auf diesen Spülschritt aber verzichten, wenn die Spurenverunreinigungen nur sehr gering sind und die dadurch hervorgerufene Kontamination des Adsorptionsmittels in vertretbaren Grenzen bleibt, oder man verwendet einen speziellen Vorfilter für die Spurenverunreinigungen, insbesondere einen Trockner für Wasserdampf im Einsatzgasgemisch (Rohgas). In jedem Fall muss das Adsorptionsmittel — in an sich bekannter Weise — hinsichtlich seiner Adsorptionseigenschaften auf die Produktgaskomponente(n) abgestimmt sein.

Es ist allgemein schon vor der Erfindung bekannt gewesen, Gasgemische unter Ausnutzung der unterschiedlichen Adsorptions- und Desorptions-Eigenschaften an Adsorptionsmitteln durch Druckwechsel oder Temperaturwechsel zu trennen. Der Erfindung liegt demgegenüber eine Arbeitsweise zugrunde, mit der es gelingt, dass vor Beginn der Desorptionsphase praktisch keine anderen Gasbestandteile ausser der (den) Produktgaskomponente(n) im Adsorber vorhanden sind.

Diese vollständige Beladung mit der Produktgaskomponente wird bei der Erfindung also einerseits dadurch erreicht, dass nach der Adsorptionsphase der Adsorber mit einem Teil des Produktgases, das bevorzugt über 99,5 Vol.-% Produktgaskomponente enthält, gespült wird und das dabei den Adsorber verlassende, an Produktgaskomponente angereicherte Gas (Rücklaufgas), dessen Produktgaskomponente-Konzentration zwischen dem Ausgangsgas und dem Produktgas liegt, erneut dem Druckwechselprozess zugeführt wird. Für zumindest das Ende der Spülphase wird gemäss der Erfindung ein Anteil des Produktgases verwendet. Dieser sollte über etwa 10 Vol.-% sein und etwa 90 Vol.-% des Produktsgases nicht überschreiten. Die Höhe des Anteiles hängt von der Konzentration der Produktgaskomponente(n) im Ausgangsgas ab; er ist desto höher, je niedriger die Produktgaskomponenten-Konzentration hier ist. Ein vorteilhaftes, weiteres Merkmal der Erfindung besteht darin, dass während der Spülung des Adsorbers mit Produktgas in den ersten zwei Dritteln der Spülphase das den Adsorber verlassende Gas in das Abgas gegeben wird und nur das im letzten Drittel anfallende, an Produktgaskomponenten angereicherte Gas (Rücklaufgas) zur erneuten Adsorption oder Spülung in einem nachfolgenden Zyklus eingesetzt wird; hierdurch wird bei hoher Ausbeute die Zykluszeit vermindert.

In besonderen Fällen kann es sich als ausreichend erweisen, die Spülung nicht bis zum vollständigen Duchbruch der Produktgaskomponente zu betreiben. Dann wird das bei der Desorption des Adsorbers gewonnene Gasgemisch zu Beginn der Desorptionsphase zum Spülen eines anderen Adsorbers verwendet, und erst nach einiger Zeit wird das in einer Reinheit von über 99,5 Vol.-% gewonnene Produktgas in einen Anteil zum Spülen und in einen in die Produktgasleitung gehenden Teilstrom aufgeteilt.

Ausserdem ist es für die gewünschte vollständige Beladung mit der (den) Produktgaskomponente(n) wichtig, dass der Druckaufbau bei Ausgangsgasen mit z.B. weniger als etwa 15 Vol.-% Kohlendioxid oder weniger als etwa 10 Vol.-% Methan mit Ausgangsgas selbst, dagegen bei Ausgangsgasen mit höherer Konzentration der Produktgaskomponente(n) mit dem Abgas der Gasgemischtrennung durchgeführt wird. Ausserdem wird nach einem weiteren, bevorzugten Merkmal der Erfindung in der Adsorptionsphase nach dem Druckaufbau nur für eine begrenzte Zeitspanne das zu trennende Ausgangsgas, später dagegen ein an der Produktgaskomponente angereichertes Gas, aus der Spülphase eines anderen Zyklus oder eine Mischung aus beiden eingeleitet. Ziel aller vorgenannten Massnahmen ist es, dass bei der Druckentspannung oder Evakuierung — die Adsorption wird vorteilhaft auf dem natürlich vorhandenen Druckniveau des Ausgangsgases vorgenommen — in der Desorptionsphase ein Produktgas mit vorzugsweise mindestens 99,5 Vol.-% Produktgaskomponente aus dem Adsorber austritt.

Die Spülung des Adsorbers sollte bei der beschriebenen Arbeitsweise im Gleichstrom zur Adsorption erfolgen, dagegen kann die Desorption des Adsorbers im Gleich- oder Gegenstrom zur Adsorption ader auch, besonders schnell, von beiden Enden des Adsorbers gleichzeitig erfolgen.

Die Zeitdauer für einen vollständigen Adsorptions- und Desorptionsschritt sollte nach der Erfindung etwa so aufgeteilt sein, dass der Druckaufbau und die Beladung des Adsorptionsmittels mit Produktgaskomponente in der Adsorptionsphase etwa zwischen halb bis doppelt so lang wie die Spülphase oder die Desorptionsphase dauern. Es hat sich nämlich gezeigt, dass beispielsweise in einer Vier-Adsorber-Anlage die Adsorptionsphase etwa doppelt so lang ist, wie die Spül- oder Desorptionsphase. In einer Drei-Adsorber-Anlage genügen in der Regel zwar gleiche Zeiten für alle drei Phasen, jedoch ist der Wirkungsgrad niedriger als bei der Vier-Adsorber-Anlage.

Für die «Spülung» eines Adsorbers wird eine relativ hohe Gasmenge benötigt, die beim Evakuieren eines anderen Adsorbers gewonnen wird. Dies bedeutet, dass von dem beim Evakuieren eines Adsorbers gewonnenen Gas nur ein Teil als Produktgas genutzt werden kann, während der andere für die Spülung verwendet werden muss. Der Energieaufwand für den Betrieb der Anlage wird wesentlich bestimmt durch die Pumpenenergie beim Evakuieren. Diese hängt ab von den Anteilen der Produktgas- zur Spülgasmenge (d.h. je weniger Spülgas im Verhältnis zum Produktgas benötigt wird, desto geringer ist der Energieverbrauch) und vom Wirkungsgrad der Vakuumpumpe über den gesamten Druckbereich. Der Erfindung liegt deshalb die weitere Aufgabe zugrunde, den Energieverbrauch und ggf. die spezifische Anlagengrösse zu verringern.

Als besonders vorteilhaft hat es sich dabei herausgestellt, zwei Vakuumpumpen einzusetzen (Version 1), von denen die erste zwischen dem Spüldruck und einem Zwischendruck und die zweite zwischen dem Zwischendruck und dem Enddruck der Evakuierung betrieben wird. Mit der ersten Pumpe wird das Spülgas gefördert, mit der zweiten das Produktgas. Bei Versuchen hat sich als besonderer Vorteil gezeigt, dass beide Pumpen während des jeweiligen Evakuierungsvorgangs nur einen Druckbereich zu durchfahren haben, der geringer ist als der zwischen Spüldruck und Enddruck des Vakuums. Es zeigt sich nämlich, dass während des ersten Evakuierungsschritts relativ viel Gas mit geringem Differenzdruck, während des zweiten Evakuierungsschrittes relativ wenig Gas bei grösserem Differenzdruck anfällt. Für beide Anforderungen gibt es Pumpen, die mit höherem Wirkungsgrad arbeiten als eine einzige Pumpe, die über den gesamten Bereich arbeitet. Als besonderer unerwarteter Vorteil dieser Vorgehensweise hat es sich herausgestellt, dass sich durch Verwendung zweier geeigneter Pumpen der Rohgas-Durchsatz durch eine Druckwechselanlage gegebener Grösse nicht unerheblich steigern lässt, da für die Desorption bei Unterdruck offensichtlich mehr Zeit benötigt wird, als die Theorie [vgl. E. Richter, u.a.; – Chemie-Ingenieur-Technik 50 (1978) S. 600/611] voraussagt.

Diese betrachtet die Desorption als Umkehrung der Adsorption, deren Geschwindigkeit allein durch die Diffusion der Gase in den Poren des Adsorbens bestimmt sit. Als weiterer Vorteil der Verwendung zweier Vakuumpumpen für die Desorption hat es sich herausgestellt, dass es nicht erforderlich ist, dass zum Spülen des Adsorbers ein Gas verwendet wird, das Produktgasqualität aufweist. Damit kann die Spülgasmenge verringert werden, ohne dass die Produktgasqualität beeinträchtigt wird. Dies hat den wesentlichen Vorteil, dass die Leistung der Druckwechselanlage durch zeitweilige Veränderungen in der Rohgaszusammensetzung weniger beeinträchtigt wird. Ein weiterer Vorteil besteht darin, dass die Pumpen weniger durch Drucksprünge beansprucht werden und dass der Mengenstrom des Produktgases über dem jeweiligen Evakuierungsvorgang weniger stark mit der Zeit schwankt. Darüber hinaus lässt sich mit der beschriebenen Version ein niedrigerer Enddruck bei der Evakuierung (da die Evakuierungsdauer verlängert wird und die Produktgas erzeugende Pumpe speziell ausgelegt sein kann) erreichen. Damit kann eine höhere Ausbeute an der(n) zu gewinnenden Komponente(n) erreicht werden.

In vielen Fällen liegt das Rohgas bei einem Druck vor, der wesentlich über Umgebungsdruck liegt. Dann ist es nicht möglich, dass das bei der Evakuierung anfallende Gas direkt für eine Spülung des Adsorbers auf dem Niveau des Beladungsdrucks zu verwenden, da Vakuumpumpen nur gegen einen begrenzten Überdruck fördern können. Damit wäre ein zusätzlicher Kompressor erforderlich, der neben erhöhten Investitionskosten auch zusätzliche Betriebskosten erfordern würde. Den Beladungsdruck abzusenken wäre eine Möglichkeit. Als besonders vorteilhaft hat es sich aber erwiesen, den Beladungsdruck auf dem höheren Druckniveau zu belassen und die Spülung des Adsorber nicht bei Beladungsdruck sondern auf einem Druckniveau durchzuführen, das von der Vakuumpumpe erreicht wird (dies gilt gleichermassen für die Modifikationen mit einer und mit zwei Vakuumpumpen). Dabei hat es sich als vorteilhaft herausgestellt, nach Abschluss der Beladung den Adsorber im Gleichstrom zu entspannen (s. Version 4).

Diese Entspannung hat bereits zur Folge, dass die zu gewinnende(n) Komponente(n) im Absorber relativ zu den anderen Komponenten angereichert wird (werden). Überraschenderweise hat es sich bei Versuchen herausgestellt, dass sich die Verluste an Produktgas, die bei der Entspannung erwartet wurden, vermeiden lassen, wenn die Beladung zu einem Zeitpunkt abgebrochen wird, an dem die Durchbruchsfront noch nicht bis zum Adsorberausgang vorgerückt ist und wenn die Entspannung im Gleichstrom in der Weise erfolgt, dass bei Beendigung der Entspannung noch kein vollständiger Durchbruch des Produktgases erfolgt ist. Überraschenderweise kann bei dieser Vorgehensweise die Spülgasmenge reduziert werden.

Unerwarteterweise wurde nun festgestellt, dass sich auch nach einer Beladung bei einem Druck der nicht wesentlich über Umgebungsdruck liegt die Spülgasmenge reduzieren lässt, wenn ein Druckausgleich mit einem Adsorber erfolgt, dessen Evakuierung gerade abgeschlossen ist und in dem noch der Enddruck der Evakuierung herrscht (Version 3). Als besonderer Vorteil zeigt es sich dabei, dass weniger Abgas für den Druckaufbau erforderlich ist. Dies ist gerade dann wichtig, wenn die zu gewinnende(n)

Komponente(n) bereits in relativ hohen Volumenanteilen (z.B. über 60 Vol.-%) vorliegt. Eine Verringerung der Spülgasmenge ergibt sich gleichermassen, wenn der Durckausgleich nach der Spülung erfolgt (Version 2). Dies wird auch beobachtet, wenn die Beladung bei erhöhtem Druck und anschliessend eine Gleichstromentspannung erfolgt.

Weitere Einzelheiten des erfindungsgemässen Verfahrens und der hierfür erforderlichen Anlage zur Durchführung des Verfahrens werden anhand der Zeichnung näher erläutert. Es zeigen:

Fig. 1 eine Anlage zur Durchführung des Verfahrens mit vier Adsorbern (Version 1);

Fig. 2 eine graphische Darstellung des Steuerablaufplanes der Phasen in den vier Adsorben der Fig. 1;

Fig. 3 eine weitere Anlage zur Durchführung des Verfahrens mit vier Adsorbern (Version 2);

Fig. 4 eine graphische Darstellung des Steuerablaufplanes der Phasen in den vier Adsorbern der Fig. 3;

Fig. 5 eine weitere Anlage zur Durchführung des Verfahrens mit vier Adsorbern (Version 3);

Fig. 6 eine graphische Darstellung des Steuerablaufplanes der Phasen in den vier Adsorbern der Fig. 5;

Fig. 7 eine weitere Anlage zur Durchführung des Verfahrens mit vier Adsorbern (Version 4);

Fig. 8 eine graphische Darstellung des Steuerablaufplanes in den vier Adsorbern der Fig. 7.

*Version 1*

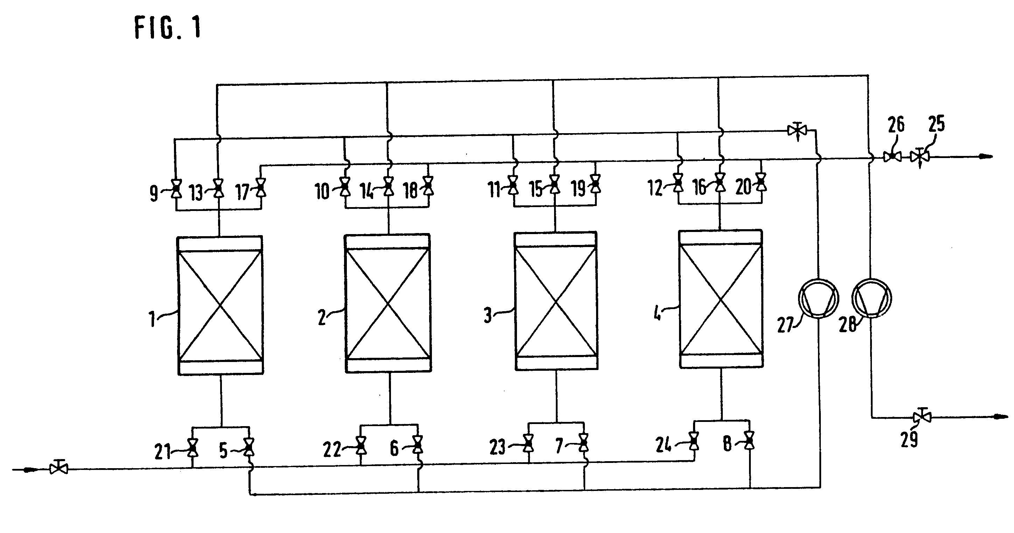

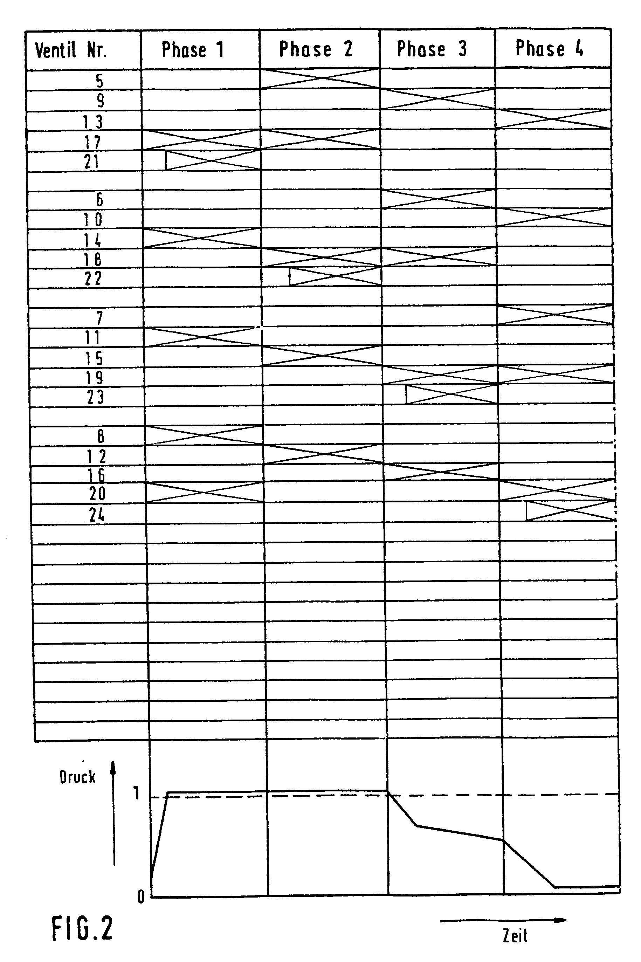

Bei dieser Version werden zwei Vakuumpumpen eingesetzt, von denen die eine Vakuumpumpe ein Spülgas absaugt und die zweite Vakuumpumpe das Produktgas absaugt. Die Trennung der Funktionen beider Vakuumpumpen bietet den besonderen Vorteil, dass jede der Pumpen spezifisch für die besondere Funktion ausgelegt werden kann. Dies bedeutet, dass die Vakuumpumpe, die zur Spülgaserzeugung eingesetzt wird, nur ein geringeres Vakuum ziehen muss als die Pumpe, die zur Produktgaserzeugung verwendet wird. Darüber hinaus müssen Spüldruck und Produktgasdruck nicht mehr identisch sein, sondern können in Grenzen, die durch die Auslegung der jeweiligen Vakuumpumpe festgelegt sind, betrieblichen Erfordernissen angepasst werden. Das Fliessbild der Anlage ist in Fig. 1 dargestellt, den Steuerablaufplan gibt Fig. 2 wieder; in letzterer ist auch der Verlauf des Drucks in Adsorber 1 über die Zeit während eines vollständigen Druckwechselzyklus eingetragen.

Das Einsatzgas liegt bei einem Druck vor, der leicht über dem Umgebungsdruck liegt, so dass der Druckverlust in den Adsorbern während der Durchströmung überwunden werden kann. Das Produktgas wird bei einem Druck gewonnen, der je nach Vakuumpumpe mehr oder weniger über dem Umgebungsdruck liegt. Nachfolgend wird die Funktionsweise der Anlage über einen vollständigen Druckwechselzyklus erklärt. Die Beschreibung beschränkt sich auf einen Adosrber, da die anderen Adsorber zeitlich versetzt dieselben Schritte durchlaufen.

Das Einsatz-(Roh)Gas strömt während des Beladungsschrittes bei Rohgasdruck über Ventil 21 durch den Adsorber 1 und über die Ventile 17 und 26 in die Abgasleitung. Die Beladung wird abgebrochen, bevor das stärker adsorbierte Gas in merklichen Konzentrationen den Adsorber verlässt. Das bei der Adsorption abströmende Gas wird gleichzeitig zum Teil auch für den Druckaufbau in Adsorber 2 verwendet. Nach Beendigung der Beladung wird Adsorber 1 mit einem Gas gespült, das stärker mit der stärker adsorbierenden Komponente angereichert ist als das Rohgas. Dazu wird bei geöffnetem Ventil 12 Gas aus Adsorber 4 mit Hilfe der Vakuumpumpe 27 abgesaugt und über Ventil 5 durch Adsorber 1 gedrückt.

Während des Spülvorgangs verlässt ein Gas den Adsorber 1 über Ventil 17, das weitgehend frei von der stärker adsorbierenden Komponente ist. Dieses Gas gelangt in die Abgasleitung. Mit dem Spülvorgang reichert sich die stärker adsorbierende Komponente in Adsorber 1 so stark an, dass bei der nachfolgenden Evakuierung mittels Pumpe 27 ein Gas gewonnen wird, dessen Gehalt an der stärker adsorbierenden Komponente nicht wesentlich unter dem Gehalt dieses Gases im Produktgas liegt. Das Gas wird bei geöffnetem Ventil 9 aus Adsorber 1 abgesaugt und bei geöffneten Ventilen 6 und 18 zum Spülen von Adsorber 2 verwendet. Dabei wird der Druck in Adsorber 1 von Rohgasdruck auf einen Druck abgesenkt, der zwischen dem Rohgasdruck und dem Endvakuum liegt, das bei der nachfolgenden zweiten Evakuierung erreicht wird. Nach Beendigung dieses ersten Evakuierungsschrittes beginnt der zweite Evakuierungsschritt, der mit der Gewinnung von Produktsgas identisch ist. Dazu wird bei geöffnetem Ventil 13 mit Vakuumpumpe 28 ein Gas aus dem Adsorber 1 abgesaugt, das Produktgasqualität aufweist. Der Druckwechselzyklus wird abgeschlossen, indem nach Beendigung der Produktion ein Druckaufbau mit Abgas erfolgt. Dazu ist an Adsorber 1 das Ventil 17 geöffnet.

Versuchsdaten:

Adsorptionsmittel: Kohlenstoffmolekularsieb mit einer BET-Oberfläche von 1100 m²/g

Beladungsdruck: 1,05 bar

Einsatzgas: 16,7 Vol.-% $CO_2$
83,3 Vol.-% $N_2$

Adsorbervolumen: 1 m³

Vorgehensweise: Version 1

Gasvolumina im Normzustand

|  | $CO_2$ (m³) | $N_2$ (m³) | Gesamt-gas (m³) |
|---|---|---|---|
| 1. Druckaufbau (Einsatzgas) | 0,25 | 1,32 | 1,57 |
| 2. Adsorption (Einsatzgas) | 3,25 | 16,94 | 20,19 |
| 3. Spülung (Produktgas 1) | 9,57 | 0,57 | 10,14 |
| 4. Desorption (Produktgas 2) | 3,19 | —— | 3,19 |

|  | $CO_2$ (m³) | $N_2$ (m³) | Gesamt-gas (m³) |
|---|---|---|---|
| Abgas: (1,67 Vol.-% $CO_2$) | 0,31 | 18,26 | 18,57 |

Ausbeute = (Produktsgasmenge) : (Einsatzgasmenge) = 0,91

*Version 2*

Bei dieser Version wird eine Vakuumpumpe eingesetzt. Die Beladung erfolgt bei einem Druck, der nahe Umgebungsdruck liegt und um einen Differenzdruck über dem Abgasdruck liegt, der dem Druckverlust in der Anlage während der Beladung entspricht. Es erfolgt ein Druckausgleich (nach erfolgter Adsorptionsspülung) in den Unterdruckbereich hinein, wodurch in besonders vorteilhafter Weise die stärker adsorbierende Komponente im Adsorber angereichert wird und die Vakuumpumpe weniger stark durch Drucksprünge beim Umschalten von einem Adsorber auf den nächsten beansprucht wird. Gleichermassen wird dadurch der Anfall des Produktgases über die Zeit vergleichmässigt.

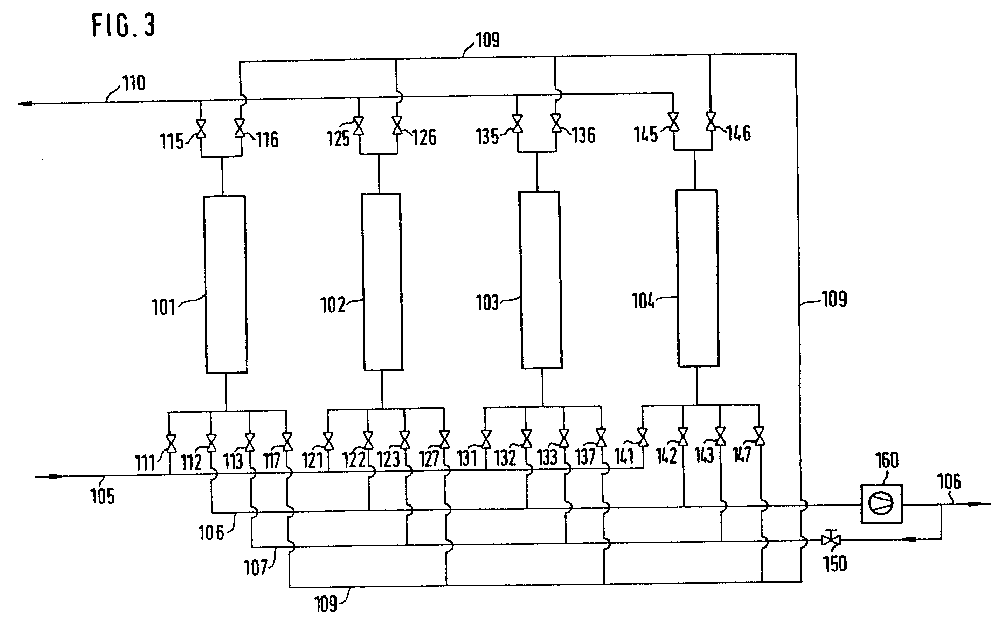

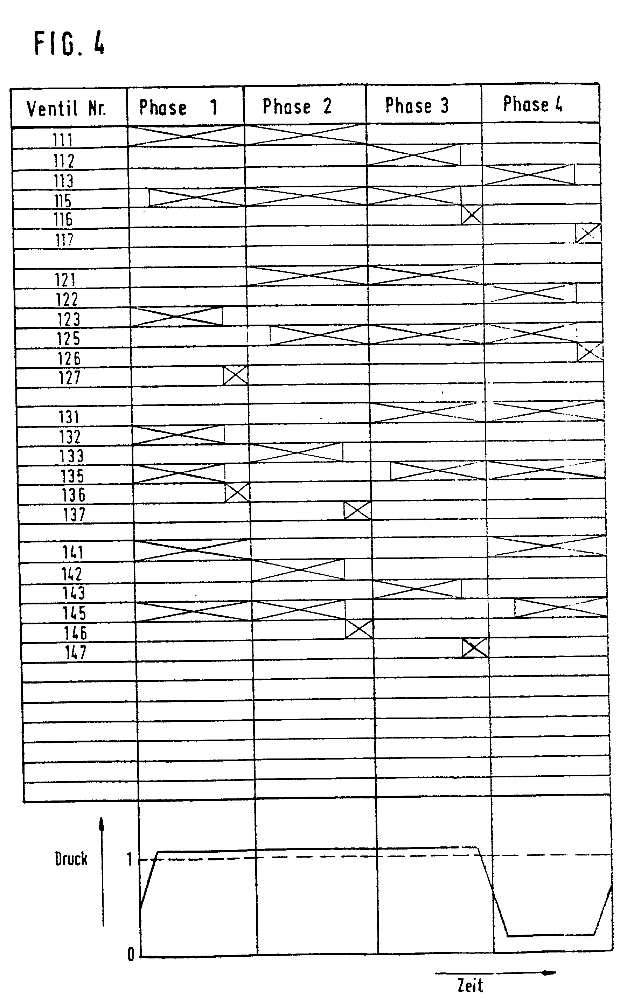

Das Fliessbild der Anlage ist in Fig. 3 dargestellt. Den Steuerablaufplan zeigt Fig. 4 in der auch der Verlauf des Drucks in Adsorber 101 während eines vollständigen Druckwechselzyklus dargestellt ist.

Nachfolgend wird die Funktionsweise der Anlage am Durchlaufen der verschiedenen Schritte während eines Druckwechselzyklus von Adsorber 101 beschrieben. Die anderern Adsorber mit ihren entsprechenden Ventilen durchlaufen zeitlich versetzt dieselben Schritte wie Adsorber 101. Dies ist direkt aus Fig. 4 zu ersehen.

Das Rohgas (Einsatzgas) strömt während des Beladungsvorgangs über die Rohgasleitung 105 sowie das Ventil 111 in den Adsorber 101. Das weitgehend von der stärker adsorbierenden Komponente befreite Abgas strömt über Ventil 115 in die Abgasleitung 110. Die Beladung wird zu einer vorgegebenen Zeit abgebrochen, bei der das stärker adsorbierende Gas noch nicht verstärkt in die Abgasleitung gelangt. Nachfolgend wird Adsorber 101 mit einem Gas gespült, das mit Hilfe der Vakuumpumpe 160 aus Adsorber 104 abgesaugt wird. Während dieses Vorgangs sind die Ventile 143, 112 und 115 geöffnet. Das Gas hat praktisch Produktgasqualität, wenn es in Adsorber 101 eintritt. Diesen verlässt ein Gas, dessen Gehalt an der stärker adsorbierenden Komponente unter dem des Rohgases liegt. Nach Abschluss der Spülung nach einer vorgegebenen Zeit wird der Druck im Adsorber in zwei Stufen abgesenkt. In der ersten Stufe erfolgt der Druckausgleich mit dem evakuierten Adsorber 104 über die geöffneten Ventile 116 und 147. Dabei strömt ein Gas über Leitung 109 von Adsorber 101 in Adsorber 104, dessen Gehalt an der stärker adsorbierenden Komponente im Mittel über dem Gehalt im Rohgas liegt. Dieser Druckausgleich wird nach einer vorgegebenen Zeit abgebrochen. Dann hat sich in beiden Adsorbern ein annähernd gleicher Druck eingestellt, der zwischen dem Rohgasdruck und dem bei der Evakuierung erreichten Enddruck liegt. Im folgenden Schritt wird Adsorber 101 durch Öffnen von Ventil 112 über Leitung 106 mit der Vakuumpumpe 160 verbunden.

Dabei sinkt der Druck mit der Zeit bis auf einen Enddruck ab. Aus dem Adsorber wird ein Gas abgesaugt, das Produktgasqualität aufweist. Dieses Gas gelangt aus Vakuumpumpe 160 zu einem Teil in die Produktgasleitung 106, zum anderen Teil wird es zum Spülen von Adsorber 102 verwendet, in den es über das Drosselventil 150, Leitung 107 und Ventil 123 gelangt. Nach Beendigung der Evakuierung von Adsorber 101 wird zu einer vorgegebenen Zeit mit der Erhöhung des Drucks in diesem Adsorber auf Beladungsdruck begonnen. Diese Druckerhöhung erfolgt in zwei Stufen. In der ersten Stufe erfolgt ein Druckausgleich mit Adsorber 102 bei geöffneten Ventilen 117 und 126 über Leitung 109. In der zweiten strömt Rohgas bei geöffnetem Ventil 111 in den Adsorber. Bei letzterem Schritt erhöht sich der Druck von dem bereits erwähnten Zwischendruck wieder auf Rohgasdruck. Danach wird wieder mit der Beladung in der bereits oben beschriebenen Weise begonnen.

Versuchsdaten:

| Adsorptionsmittel: | Kohlenstoffmolekularsieb mit einer BET-Oberfläche von 1100 m²/g |
| Beladungsdruck: | 1,05 bar |
| Einsatzgas: | 16,7 Vol.-% $CO_2$ 83,3 Vol.-% $N_2$ |
| Adsorbervolumen: | 1 m³ |
| Vorgehensweise: | Version 2 |

Gasvolumina im Normzustand

| | $CO_2$ (m³) | $N_2$ (m³) | Gesamtgas (m³) |
|---|---|---|---|
| 1. Druckaufbau (Einsatzgas) | 0,29 | 1,45 | 1,74 |
| 2. Adsorption (Einsatzgas) | 3,79 | 18,90 | 22,69 |
| 3. Spülung (Produktgas) | 10,67 | —— | 10,67 |
| 4. Desorption (Produktgas) | 14,20 | —— | 14,20 |
| Nettoproduktgas (99,9 Vol.-% $CO_2$) | 3,53 | —— | 3,53 |
| Abgas: (2,63 Vol.-% $CO_2$) | 0,55 | 20,35 | 20,90 |

Ausbeute = (Produktsgasmenge) : (Einsatzgasmenge) = 0,87

*Version 3*

Bei dieser Version ist auch nur eine Vakuumpumpe erforderlich. Es wird in besonders vorteilhafter Weise ausgenutzt, dass nach Beendigung des Spülvorgangs die Konzentration der stärker adsorbierenden Komponente im Adsorber noch weiter erhöht werden kann, so dass weniger Spülgas erforderlich ist oder ein Gas mit einer höheren Reinheit gewonnen werden kann. Ferner wird die Vakuumpumpe mit weniger starken Drucksprüngen belastet, da zu Beginn der Evakuierung bereits ein Druck vorliegt, der unter dem Rohgasdruck ist. Dadurch gelingt es zusätzlich, die über den Evakuierungsvorgang anfallenden Produktgasstrom zu vergleichmässigen.

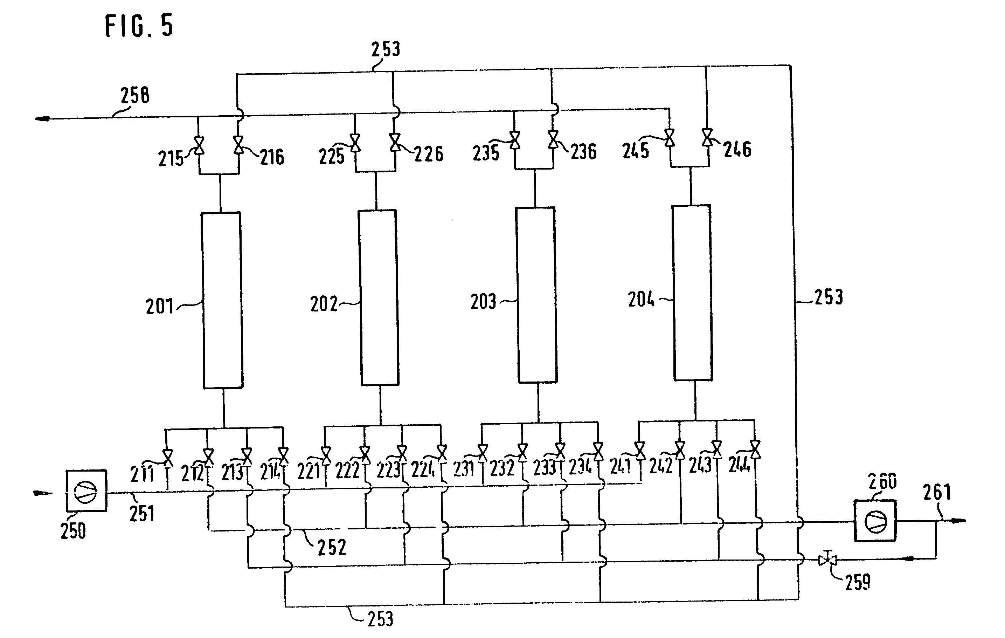

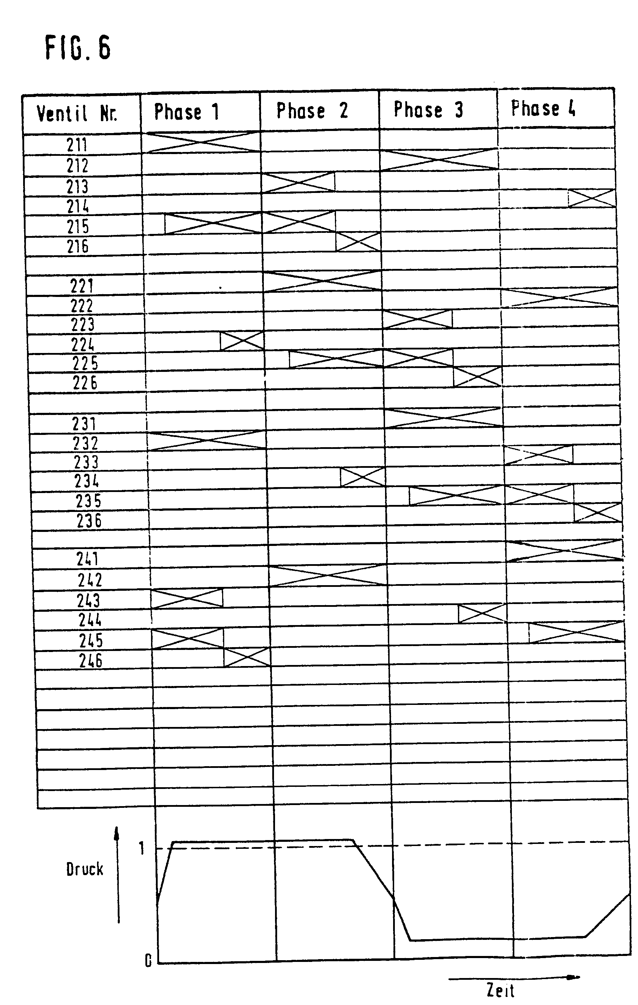

Das Fliessbild der entsprechenden Anlage ist in Fig. 5 dargestellt, der Steuerablaufplan in Fig. 6. In letzterer ist auch der Verlauf des Drucks in Adsorber 201 über einen vollständigen Druckwechselzyklus dargestellt.

Das Einsatzgas liegt bei einem Druck vor, der leicht über dem Umgebungsdruck liegt, so dass unter Berücksichtigung des Druckverlusts in der Anlage das Abgas mit Umgebungsdruck abströmen kann. Diese Festlegung des Drucks ist allerdings nicht zwingend für die Verfahrensweise. Das Produktgas wird bei einem Druck gewonnen, der je nach Vakuumpumpe mehr oder weniger über dem Umgebungsdruck liegt. Nachfolgend wird die Funktionsweise der Anlage über einen vollständigen Druckwechselzyklus erklärt. Die Beschreibung beschränkt sich auf Adsorber 201, da die anderen Adsorber 202-204 zeitlich versetzt dieselben Schritte durchlaufen.

Das Rohgas (Einsatzgas) strömt über Leitung 251 bei geöffnetem Ventil 211 in den Adsorber 201, in dem Rohgasdruck vorliegt. Die stärker adsorbierende Komponente wird während dieses Beladungsvorgangs weitgehend adsorbiert, so dass über Ventil 215 ein so gut wie vollständig von der adsorbierenden Komponente befreites Gas in die Abgasleitung 258 strömt.

Die Beladung wird beendet, bevor die Konzentration der stärker adsorbierenden Komponente im Abgas merklich ansteigt. Danach wird der Adsorber gespült mit einem Gas, das während der Evakuierung von Adsorber 204 gewonnen wird. Dazu ist an Adsorber 204 das Ventil 242 geöffnet. Das Gas wird über die Vakuumpumpe 260, das Drosselventil 259 bei geöffnetem Ventil 213 durch Absorber 201 gedrückt. Über das geöffnete Ventil 215 gelangt ein Gas in die Abgasleitung 258, dessen Gehalt an der zu gewinnenden, stärker adsorbierenden Komponente geringer ist als im Rohgas. Nach Abschluss dieses Spülvorgangs, der bei Rohgasdruck abläuft, erfolgt ein Druckausgleich zwischen Adsorber 201 und dem evakuierten Adsorber 203. Dazu sind die Ventile 216 und 234 geöffnet. Während des Druckausgleichvorgangs wird ein Gas aus Adsorber 201 in Adsorber 203 gesaugt, dessen Konzentration an der stärker adsorbierenden Komponente noch wesentlich unter der Rohgaskonzentration liegt. Im nächsten Schritt wird der Adsorber 201 evakuiert von einem Druck aus, der zwischen dem Rohgasdruck und dem Enddruck der nachfolgenden Evakuierung liegt. Während der Evakuierung ist Ventil 212 geöffnet. Das Gas gelangt über Leitung 252 durch die Vakuumpumpe 260 in die Produktgasleitung 261. Ein Teil des Gases kann während der Gesamtdauer der Evakuierung oder während eines Teils der Evakuierungsdauer zum Spülen von Adsorber 202 verwendet werden, wozu Ventil 223 und 225 an Adsorber 202 geöffnet sind. Bei der Evakuierung sinkt der Druck im Adsorber 201 von dem bereits erwähnten Zwischendruck auf einen Enddruck ab, der zwischen 10 und 600 mbar je nach Anwendung und Auslegung der Pumpe 260 liegen kann. Nach Beendigung der Evakuierung, die mit der Produktion identisch ist, erfolgt nach einer Totzeit der Druckaufbau in Adsorber 201 in zwei Schritten. Im ersten Schritt wird ein Druckausgleich des Adsorbers 201 mit Adsorber 203 durchgeführt, wozu die Ventile 214 und 236 geöffnet sind. Von dem sich dabei einstellenden Zwischendruck aus erfolgt der zweite Teil des Druckaufbaus mit Rohgas. Dieses strömt über Leitung 251 bei geöffnetem Ventil 211 in Adsorber 201. Danach beginnt der Druckwechsel von neuem.

Versuchsdaten:

Adsorptionsmittel:   Kohlenstoffmolekularsieb mit einer BET-Oberfläche von $1100 \ m^2/g$

Beladungsdruck:   1,05 bar

Einsatzgas:   16,7 Vol.-% $CO_2$   83,3 Vol.-% $N_2$

Adsorbervolumen:   $1 \ m^3$

Vorgehensweise:   Version 3

Gasvolumina im Normzustand

| | $CO_2$ $(m^3)$ | $N_2$ $(m^3)$ | Gesamtgas $(m^3)$ |
|---|---|---|---|
| 1. Druckaufbau (Einsatzgas) | 0,32 | 1,60 | 1,92 |
| 2. Adsorption (Einsatzgas) | 3,99 | 19,90 | 23,89 |
| 3. Spülung (Produktgas) | 8,07 | —— | 8,07 |
| 4. Desorption (Produktgas) | 12,18 | —— | 12,18 |
| Nettoproduktgas (99,9 Vol.-% $CO_2$) | 4,11 | —— | 4,11 |
| Abgas: (0,90 Vol.-% $CO_2$) | 0,20 | 21,50 | 21,70 |

Ausbeute = (Produktsgasmenge) : (Einsatzgasmenge) = 0,95

*Version 4*

Bei dieser Version wird eine Vakuumpumpe eingesetzt. Die Beladung erfolgt bei erhöhtem Druck, wobei das Rohgas bereits bei erhöhtem Druck vorliegen kann bzw. auf diesem Druck mittels vorgeschaltetem Kompressor zu verdichten ist. Die Adsorption auf erhöhtem Druck bringt den besonderen Vorteil mit sich, dass die Baugrösse der Adsorber verringert werden kann, da mehr Gas pro Volumeneinheit Adsorptionsmittel adsorbiert werden kann. Ferner ermöglicht der nachfolgend beschriebene Entspannungsschritt im Gleichstrom zur Adsorption eine weitere gezielte Anreicherung der stärker adsorbierenden Komponente im Adsorber, so dass ein Spülschritt, wie er bei den Versionen 1 bis 3 erforderlich war, hier unter Umständen entfallen kann.

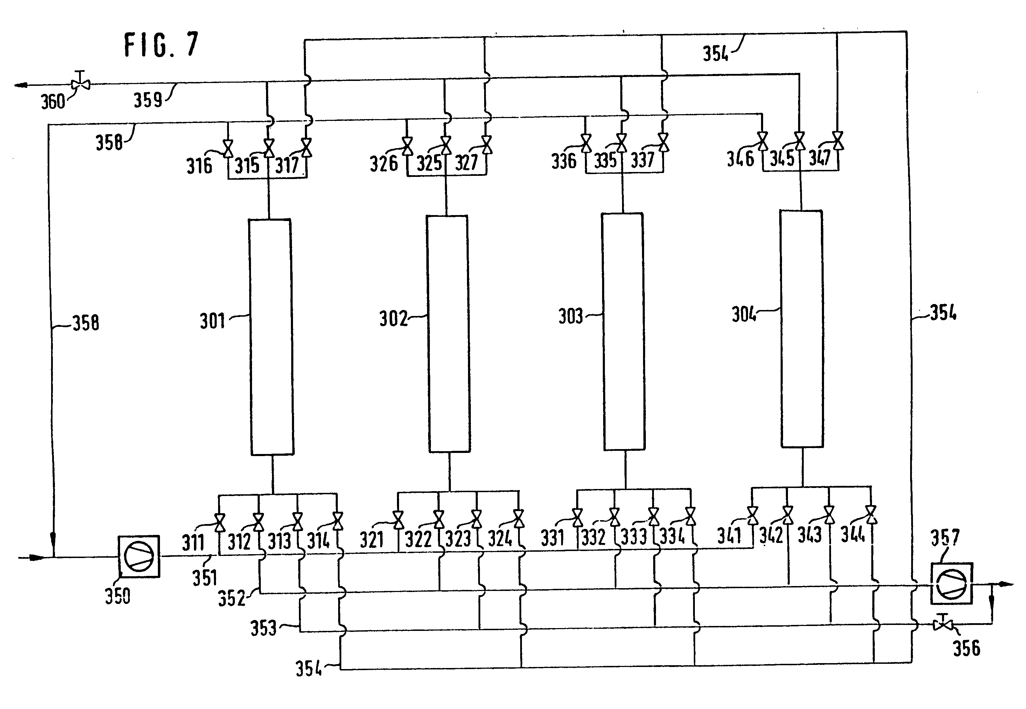

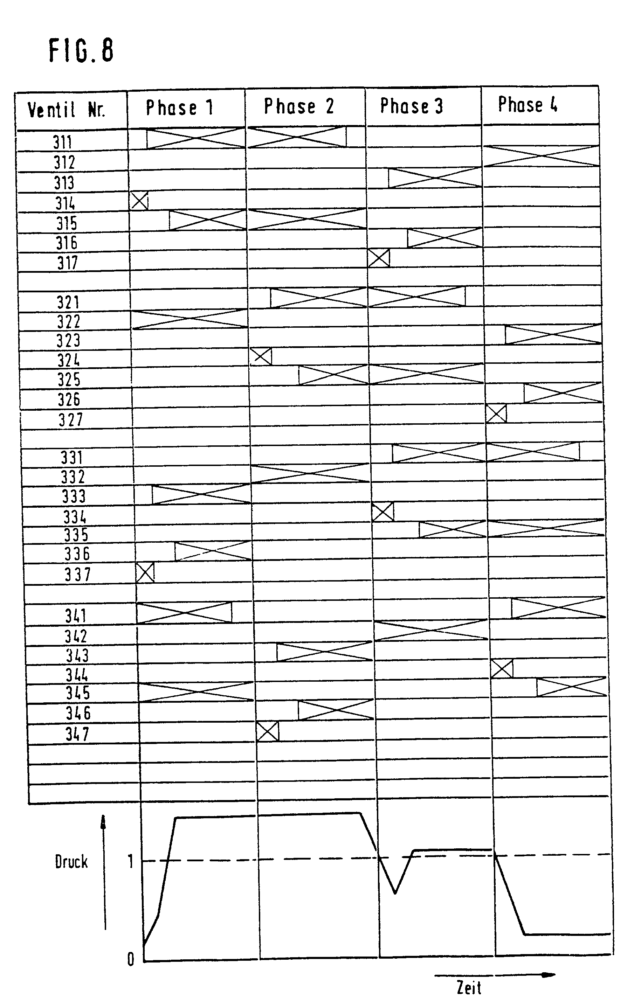

Das Fliessbild der Anlage ist in Fig. 7 dargestellt, den Steuerablaufplan zeigt Fig. 8, in der auch der Verlauf des Drucks in Adsorber 301 während eines vollständigen Druckwechselzyklus dargestellt ist.

Das Einsatzgas liegt bei einem Druck vor, der über dem Umgebungsdruck liegt. Vorzugsweise wird der Druck über 2 bar liegen. Das Produktgas wird bei einem Druck gewonnen, der je nach Vakuumpumpe mehr oder weniger über dem Umgebungsdruck liegt. Nachfolgend wird die Funktionsweise der Anlage anhand der Schaltungen für Adsorber 301 über einen vollständigen Druckwechselzyklus erklärt. Die anderen Adsorber durchlaufen zeitlich versetzt dieselben Schritte.

Das Rohgas strömt während des Beladungsvorgangs über die Rohgasleitung 351 und das Ventil 311 in den Adsorber 301 und verlässt diesen über Ventil 315. Das weitgehend von der stärker adsorbierenden Komponente befreite Gas strömt über Ventil 315 in die Abgasleitung 359, in der das Drosselventil 360 eingebaut ist. Letzteres hat die Funktion, den Druck in den Adsorbern während der Beladungsschritte konstant zu halten.

Die Beladung wird einige Zeit, bevor die Konzentration der stärker adsorbierenden Komponente im Abgas merklich steigt, abgebrochen. Der Beladung schliesst sich eine Entspannung des Adsorbers 301 an, wozu bei geschlossenem Ventil 311 Ventil 316 geöffnet bleibt. Während der Entspannungsphase, die nach einer vorgegebenen Zeit oder bei Erreichen eines vorgegebenen Druckes, welcher über Umgebungsdruck liegt, abgebrochen wird, strömt ein Gas, das weitgehend frei von der stärker adsorbierenden Komponente ist, in die Abgasleitung 359. Im nächsten Schritt erfolgt ein Druckausgleich zwischen Adsorber 301 und 303 wozu die Ventile 317 und 334 geöffnet sind. Dabei senkt sich der Druck im Adsorber 301 bis auf einen Wert ab, der in der Regel unter dem Umgebungsdruck liegt. Nachfolgend kann der Adsorber 301 mit einem Gas gespült werden, das während der Evakuierung von Adsorber 304 anfällt. Der Druck während der Spülung kann bei Umgebungsdruck oder auch leicht darunter oder darüber eingestellt werden. Während der Spülphase gelangt das über Ventil 342 durch die Vakuumpumpe 357 abgesaugte Gas nach Durchströmen des Drosselventils 356 über Leitung 353 und Ventil 313 in den Adsorber 301. Aus diesem strömt ein Gas, dessen Gehalt an der stärker adsorbierenden Komponente über dem Gehalt im Rohgas liegt, über das geöffnete Ventil 316 und Leitung 358 zurück vor den Verdichter 350. Nach Beendigung der Spülung wird der Druck in Adsorber 301 abgesenkt. Dazu wird bei geöffnetem Ventil 312 über Leitung 352 Gas mit Hilfe der Vakuumpumpe 357 abgesaugt. Von diesem Gas, das Produktgasqualität aufweist, gelangt während eines Teiles der Evakuierungsdauer oder auch während der gesamten Evakuierungsdauer ein fest eingestellter Anteil des Gases über Ventil 356, Leitung 353 und Ventil 323 zum Spülen in Adsorber 302. Nach Beendigung der Evakuierungsphase, die mit der Produktion identisch ist, erfolgt der Druckaufbau auf Beladungsdruck in zwei Stufen. In der ersten Stufe erfolgt ein Druckausgleich mit Absorber 303, der im Zustand nach der Gleichstromentspannung vorliegt. Dazu sind die Ventile 314 und 337 geöffnet. Der Druckausgleich wird nach einer vorgegebenen Zeit abgebrochen. Dann hat sich in beiden Adsorbern ein Druck eingestellt, der in der Regel unter Umgebungsdruck liegt.

Der nachfolgende Druckaufbau erfolgt bei geöffnetem Ventil 311 mit Rohgas. Danach beginnt ein neuer Zyklus in der beschriebenen Art mit einer erneuten Beladung des Adsorbers.

Versuchsdaten:

| | |
|---|---|
| Adsorptionsmittel: | Kohlenstoffmolekularsieb mit einer BET-Oberfläche von 1100 m²/g |
| Beladungsdruck: | 3,0 bar |
| Einsatzgas: | 16,7 Vol.-% $CO_2$ 83,3 Vol.-% $N_2$ |
| Adsorbervolumen: | 1 m³ |
| Vorgehensweise: | Version 4 |

Gasvolumina im Normzustand

| | $CO_2$ (m³) | $N_2$ (m³) | Gesamt-gas (m³) |
|---|---|---|---|
| 1. Druckaufbau (Einsatzgas) | 1,15 | 5,76 | 6,91 |
| 2. Adsorption (Einsatzgas) | 5,93 | 29,58 | 35,51 |
| 3. Spülung (Produktgas) | 6,68 | —— | 6,68 |
| 4. Desorption (Produktgas) | 13,48 | —— | 13,48 |
| Nettoproduktgas (99,9 Vol.-% $CO_2$) | 6,80 | —— | 6,80 |
| Abgas: (0,8 Vol.-% $CO_2$) | 0,28 | 35,34 | 35,62 |

Ausbeute = (Produktsgasmenge) : (Einsatzgasmenge) = 0,96

Versuchsdaten:

| | |
|---|---|
| Adsorptionsmittel: | Silicagel |
| Beladungsdruck: | 3,0 bar |
| Einsatzgas: | 16,7 Vol.-% $CO_2$ 83,3 Vol.-% $N_2$ |
| Adsorbervolumen: | 1 m³ |
| Vorgehensweise: | Version 4 |

Gasvolumina im Normzustand

| | $CO_2$ (m³) | $N_2$ (m³) | Gesamt-gas (m³) |
|---|---|---|---|
| 1. Druckaufbau (Einsatzgas) | 0,80 | 3,98 | 4,78 |
| 2. Adsorption (Einsatzgas) | 5,23 | 26,09 | 31,32 |

| | $CO_2$ (m³) | $N_2$ (m³) | Gesamt-gas (m³) |
|---|---|---|---|
| 3. Spülung (Produktgas) | 7,14 | —— | 7,14 |
| 4. Desorption (Produktgas) | 12,51 | —— | 12,51 |
| Nettoproduktgas (99,9 Vol.-% $CO_2$) | 5,37 | —— | 5,37 |
| Abgas: (2,2 Vol.-% $CO_2$) | 0,66 | 30,07 | 30,73 |

Ausbeute = (Produktsgasmenge) : (Einsatzgasmenge) = 0,89

Vergleichsbeispiel (gemäss DE-A-3 222 560 - Beispiel 1)

| | |
|---|---|
| Adsorptionsmittel: | Kohlenstoffmolekularsieb mit einer BET-Oberfläche von 1100 m²/g |
| Beladungsdruck: | 1,2 bar |
| Einsatzgas: | 16,7 Vol.-% $CO_2$ 83,3 Vol.-% $N_2$ |
| Adsorbervolumen: | 1 m³ |

Gasvolumina im Normzustand

| | $CO_2$ (m³) | $N_2$ (m³) | Gesamt-gas (m³) |
|---|---|---|---|
| 1. Druckaufbau (Einsatzgas) | 0,85 | 4,25 | 5,10 |
| 2. Adsorption (Einsatzgas) | 4,09 | 20,40 | 24,49 |
| 3. Spülung (Produktgas) | 18,37 | —— | 18,37 |
| 4. Desorption (Produktgas) | 22,50 | —— | 22,50 |
| Nettoproduktgas (99,9 Vol.-% $CO_2$) | 4,13 | —— | 4,13 |
| Abgas: (2,2 Vol.-% $CO_2$) | 0,81 | 24,65 | 25,46 |

Ausbeute = (Produktsgasmenge) : (Einsatzgasmenge) = 0,84

**Patentansprüche**

1. Verfahren zur Abtrennung und Gewinnung von relativ stark an Adsorptionsmitteln adsorbierbaren Gasen (Produktgaskomponenten) aus ansonsten im wesentlichen nur schwächer adsorbierbare Gase (Abgaskomponenten) enthaltenden Gasgemischen an Adsorptionsmitteln mittels Druckwechseladsorptionstechnik in Adsorbern, gekennzeichnet durch die Kombination folgender Verfahrensschritte:

a) der Adsorptionsprozess erfolgt unter Verwendung von vier parallel geschalteten Adsorbern, die zeitversetzt jeweils den gleichen Adsorptionszyklus vollziehen;

b) in der Adsorptionsphase wird das zu trennende Ausgangsgemisch, das Methan oder Kohlendioxid enthält, in einem Adsorber mit einem Druck, der oberhalb Atmosphärendruck liegt, unter Adsorption des Produktgases und Abströmen des Abgases, das teilweise zum Druckaufbau in einem anderen, parallel betriebenen Adsorber verwendet wird, durch eine Schicht aus einem kohlenstoffhaltigen Adsorptionsmittel geleitet, bis das Produktgas kurz vor dem Durchbruch steht;

c) Im Anschluss an die Adsorptionsphase wird der Gasdruck im Adsorber durch Abströmen einer Teilgasmenge im Gleichstrom zur Strömungsrichtung bei der Adsorption abgesenkt und diese Teilgasmenge in einem anderen, parallel geschalteten Adsorber zum Druckaufbau oder zum Spülen verwendet.

d) in der Spülphase wird der Adsorber mit zumindest zum Ende der Spülung etwa Produktgasqualität aufweisenden Gas gespült, das einem anderen, parallel geschalteten Adsorber entnommen wird, der desorbiert wird, wobei das zunächst abströmende Gas ins Abgas und das später abströmende, an Produktgas angereicherte Gas einem anderen, parallel geschalteten Adsorber zum Druckaufbau zugeführt wird;

e) in der Desorptionsphase wird die Produktgaskomponente durch Druckerniedrigung und ggf. Spülen desorbiert und ein hoch angereichertes Produktgas erhalten (Produktgasgewinnung), von dem ein Teil zum Spülen einem anderen, parallel geschalteten Adsorber zugeführt und das übrige gewonnen wird;

f) in der Druckaufbauphase wird der Gasdruck im Adsorber teils mit einem Gasgemisch aus einem anderen, parallel geschalteten Adsorber, teils mit Ausgangsgas für eine erneute Adsorptionsphase wieder aufgebaut;

g) anschliessend beginnt der Gesamtzyklus des Adsorbers erneut mit der Adsorptionsphase.

2. Verfahren nach Anspruch 1, dadurch gekennzeichnet, dass die Absenkung des Gasdruckes im Adsorber gemäss Merkmal c) im Anschluss an die Spülphase gemäss Merkmal d) vorgenommen wird.

3. Verfahren nach einem der Ansprüche 1 und 2, dadurch gekennzeichnet, dass ein Anteil von 10 bis 90 Vol.-% des Produktgases zum Spülen verwendet wird.

4. Verfahren nach Anspruch 3, dadurch gekennzeichnet, dass das insbesondere im letzten Drittel der Spülphase abströmende, an Produktgas angereicherte Gas am Ende der Adsorptionsphase oder am Anfang der Spülphase durch die Adsorptionsmittelschicht eines anderen, parallel geschalteten Adsorbers geleitet wird.

5. Verfahren nach einem der Ansprüche 1 bis 4, dadurch gekennzeichnet, dass die Spülung des Adsorbers im Gleichstrom zur Adsorption erfolgt.

6. Verfahren nach einem der Ansprüche 1 bis 5, dadurch gekennzeichnet, dass die Desorption des Adsorbers von beiden Enden des Adsorbers gleichzeitig erfolgt.

7. Verfahren nach einem der Ansprüche 1 bis 6, dadurch gekennzeichnet, dass der Druckaufbau und die Beladung des Adsorbers in der Adsorptionsphase zusammengenommen etwa zwischen halb so lang bis doppelt so lang wie die Spülphase oder die Desorptionsphase dauern.

8. Verfahren nach einem der Ansprüche 1 bis 7, dadurch gekennzeichnet, dass die Desorption des Adsorbers in zwei Schritten durchgeführt wird, wobei im ersten Schritt eine erste Vakuumpumpe bei einem Druck, der vom Spülgasdruck auf einen Zwischendruck abfällt, ein Gas aus dem Adsorber ansaugt, welches zur Spülung eines anderen Adsorbers verwendet wird, und in einem zweiten, direkt nachfolgenden Schritt eine zweite Vakuumpumpe bei einem Druck, der von dem Zwischendruck auf den Enddruck der Evakuierung abfällt, das Produktgas aus dem Adsorber absaugt.

9. Verfahren nach Anspruch 8, dadurch gekennzeichnet, dass der Zwischendruck um einen Anteil zwischen 0,1 und 0,95, vorzugsweise zwischen 0,4 bis 0,8, der Differenz aus Spülgasdruck und Enddruck der Evakuierung oberhalb des Enddruckes liegt, und dass der Enddruck zwischen 0,01 und 0,6, vorzugsweise zwischen 0,03 und 0,2 bar liegt.

10. Verfahren nach einem der Ansprüche 1 bis 9, dadurch gekennzeichnet, dass die Spülung des Adsorbers (Spülphase) bei einem Druck erfolgt, der niedriger als der Druck während der Beladung (Adsorptionsphase) ist.

11. Verfahren nach Anspruch 10, dadurch gekennzeichnet, dass nach einer Beladung bei einem Druck, der über Umgebungsdruck, vorzugsweise über 3 bar liegt, das während der Druckabsenkung (auf den Spülgasdruck) abströmende Gas verworfen wird (Abgas), solange die stärker adsorbierende(n) [zu gewinnende(n)] Komponente(n) in Volumenanteilen vorliegt (vorliegen), die unter denen im Rohgas liegen.

12. Verfahren nach Anspruch 10, dadurch gekennzeichnet, dass nach einer Beladung bei einem Druck, der über Umgebungsdruck, vorzugsweise über 3 bar liegt, das während der Druckabsenkung (auf den Spülgasdruck) abströmende Gas zum Druckaufbau in einem anderen Adsorber verwendet wird.

13. Verfahren nach Anspruch 10 bis 12, dadurch gekennzeichnet, dass nach einer Beladung bei einem Druck, der über Umgebungsdruck, vorzugsweise über 3 bar liegt, das während der Druckabsenkung abströmende Gas vor einen Verdichter des Rohgases zurückgeführt wird, sobald die stärker adsorbierende(n) [zu gewinnende(n)] Komponente(n) in Volumenteilen vorliegt (vorliegen), die über denen im Rohgas liegen.

14. Verfahren nach einem der Ansprüche 10 bis 13, dadurch gekennzeichnet, dass der Spülgasdruck dann erreicht wird, wenn der Volumenanteil des (der) zu gewinnenden Komponente(n) im abströmenden Gas einen vorgegebenen Wert überschreitet, der zwischen dem mittleren Volumenanteil im Produktgas und dem Volumenanteil im Rohgas liegt.

**Claims**

1. A method of separating and recovering gases (product gas components) which are relatively highly adsorbable by adsorbents from gas mixtures also containing substantially only weakly adsorbable gases (waste gas components), by adsorption agents by means of pressure-charge adsorption technology, characterized by the combination of the following method steps:

a) the adsorption process takes place using four adsorbers which are arranged in parallel and which perform the same adsorption cycle with a time lag in each case;

b) in the adsorption phase the starting gas mixture which is to be separated and which contains methane or carbone dioxide is conveyed in an adsorber at a pressure above atmospheric pressure, during the adsorption of the product gas and the discharge of the waste gas which is used in part for building up pressure in another adsorber driven in parallel, through a layer of a carbon-containing adsorption agent, until the product gas is just on the point of breaking through,

c) after the adsorption phase the gas pressure in the adsorbers is reduced by discharging a partial quantity of gas in parallel flow with the direction of flow during adsorption, and this partial quantity of gas is used in another adsorber arranged in parallel for building up pressure or for rinsing;

d) in the rinsing phase the adsorber is rinsed with gas which has approximately product gas quality at least at the end of rinsing and which is removed from another adsorber which is arranged in parallel and is desorbed, the gas which first flows off being conveyed into the waste gas and the gas which subsequently flows off and which is enriched in product gas being conveyed to another adsorber arranged in parallel for building up pressure;

e) in the adsorption phase the product gas component is desorbed by a drop in pressure and, where appropriate, rinsing and a highly enriched product gas in obtained (product gas recovery), of which one part is conveyed for rinsing to another adsorber arranged in parallel and the other part is recovered;

f) in the pressure build-up phase the gas pressure in the adsorber is built up again partly with a gas mixture from another adsorber arranged in parallel and partly with starting gas for a new adsorption phase;

g) the entire cycle of the adsorber then begins again with the adsorption phase.

2. A method according to claim 1, characterized in that the lowering of the gas pressure in the absorber according to feature c) is performed subsequently to the rinsing phase according to feature d).

3. A method according to one of claims 1 and 2, characterized in that a portion of from 10 to 90% by volume of the product gas is used for rinsing.

4. A method according to claim 3, characterized in that the gas enriched with product gas and flowing off in particular in the final third of the rinsing phase is conveyed at the end of the adsorption phase or at the beginning of the rinsing phase through the adsorption agent layer of another adsorber arranged in parallel.

5. A method according to any one of claims 1 to 4, characterized in that the rinsing of the adsorber is performed in parallel flow for adsorption.

6. A method according to any one of claims 1 to 5, characterized in that the desorption of the adsorber is performed from both ends of the adsorber simultaneously.

7. A method according to any one of claims 1 to 6, characterized in that the pressure build-up and the charging of the adsorber in the adsorption phase last together approximately between half as long and double as long as the rinsing phase or the desorption phase.

8. A method according to any one of claims 1 to 7, characterized in that the desorption of the adsorber is performed in two steps, in the first of which a first vacuum pump at a pressure which drops from the rinsing gas pressure to an intermediate pressure draws a gas out of the adsorber, which gas is used for rinsing another adsorber, and in the second step, following immediately, a second vacuum pump at a pressure which drops from the intermediate pressure to the final pressure of evacuation draws the product gas out of the adsorber.

9. A method according to claim 8, characterized in that the intermediate pressure lies above the final pressure by an amount between 0.1 and 0.95, preferably between 0.4 and 0.8, of the difference between the rinsing gas pressure and the final pressure of evacuation, and the final pressure lies between 0.01 and 0.6, preferably between 0.03 and 0.2 bar.

10. A method according to any one of claims 1 to 9, characterized in that the rinsing of the adsorber (rinsing phase) is performed at a pressure which is lower than the pressure during charging (adsorption phase).

11. A method according to claim 10, characterized in that after charging at a pressure which lies above the ambient pressure, preferably above 3 bar, the gas flowing off during the drop in pressure (to the rinsing gas pressure) is ejected (waste gas), as long as the more highly adsorbing component or components (to be recovered) is or are present in parts by volume which are below those in the crude gas.

12. A method according to claim 10, characterized in that after charging at a pressure which lies above the ambient pressure, preferably above 3 bar, the gas flowing off during the drop in pressure (to the rinsing gas pressure) is used for building up pressure in another adsorber.

13. A method according to claim 10 to 12, characterized in that after charging at a pressure which lies above the ambient pressure, preferably above 3 bar, the gas flowing off during the drop in pressure is fed back upstream of a compressor of the crude gas as soon as the more highly adsorbing component or components (to be recovered) is or are present in parts by volume which are above those in the crude gas.

14. A method according to any one of claims 10 to 13, characterized in that the rinsing gas pressure is achieved when the part by volume of the component or components to be recovered in the gas flowing off exceeds a predetermined value which lies between the average part by volume in the product gas and the part by volume in the crude gas.

# Revendications

1. Procédé pour l'extraction et la séparation de produits gazeux relativement fortement adsorbables sur des produits adsorbants (composants du gaz de production) de mélanges gazeux contenant en outre essentiellement des produits gazeux plus faiblement adsorbables (composants des gaz résiduaires) sur des produits adsorbants, dans des adsorbeurs, par la technique d'adsorption par compressions et détentes alternantes, caractérisé par une combinaison des stades opératoires ci-après:

a) le processus d'adsorption est réalisé dans quatre adsorbeurs montés en parallèle, dans lesquels le même cycle d'adsorption se déroule avec un certain décalage dans le temps;

b) dans la phase d'adsorption, le mélange gazeux de départ à séparer, qui contient du méthane ou de l'anhydride carbonique, passe dans un adsorbeur, sous une pression supérieure à la pression atmosphérique, à travers une couche d'un produit adsorbant carboné, sur lequel le (les composants du) gaz à produire est (sont) adsorbé(s) et à travers lequel s'écoule le gaz résiduaire, qui est en partie utilisé pour augmenter la pression dans l'un des autres adsorbeurs montés en parallèle jusqu'au moment où le gaz de production est près de quitter la couche adsorbante;

c) après la phase d'adsorption, la pression de gaz dans l'adsorbeur est abaissée par l'évacuation d'une fraction des produits gazeux en un courant parallèle à la direction d'écoulement pendant l'adsorption, cette fraction étant utilisée dans l'un des autres adsorbeurs montés en parallèle, soit pour augmenter la pression, soit pour le rinçage;

d) dans la phase de rinçage, l'adsorbeur est traversé par un mélange gazeux qui, au moins vers la fin du rinçage, est proche de la composition du gaz à produire et qui est obtenu par désorption dans l'un des autres adsorbeurs montés en parallèle, le produit sortant d'abord étant considéré comme gaz résiduaire et le produit gazeux soutiré ensuite, enrichi en gaz à produire, étant utilisé dans l'un des autres adsorbeurs montés en parallèle pour augmenter la pression;

e) dans la phase de désorption, on provoque par un abaissement de la pression (une détente) et éventuellement un rinçage la désorption des composants du gaz de production et l'on soutire un gaz de production fortement enrichi, dont une partie est utilisée pour le rinçage de l'un des autres adsorbeurs montés en parallèle, le restant constituant le produit final;

f) dans la phase d'augmentation de la pression, la pression de gaz dans l'adsorbeur est augmentée en partie à l'aide du mélange gazeux soutiré de l'un des autres adsorbeurs montés en parallèle et en partie à l'aide du mélange gazeux de départ pour initier une nouvelle phase d'adsorption;

g) on recommence ensuite, avec la phase d'adsorption, un nouveau cycle complet dans l'adsorbeur.

2. Procédé suivant la revendication 1, caractérisé en ce que l'abaissement de la pression de gaz dans l'adsorbeur selon la caractéristique c) est réalisé à la suite de la phase de rinçage selon la caractéristique d).

3. Procédé suivant l'une des revendications 1 et 2, caractérisé en ce que l'on emploie entre 10 et 90% en volume du gaz de production pour le rinçage.

4. Procédé suivant la revendication 3, caractérisé en ce que l'on fait passer le mélange gazeux enrichi en (composants du) gaz de production, sortant de la phase de rinçage, et en particulier celui obtenu pendant le dernier tiers de celle-ci à travers la couche de produit adsorbant de l'un des autres adsorbeurs montés en parallèle, soit à la fin de la phase d'adsorption, soit au début de la phase de rinçage.

5. Procédé suivant l'une des revendications 1 à 4, caractérisé en ce que le rinçage d'un adsorbeur est réalisé en un courant parallèle à l'adsorption.

6. Procédé suivant l'une des revendications 1 à 5, caractérisé en ce que la désorption est réalisée simultanément à partir des deux extrémités de l'adsorbeur.

7. Procédé suivant l'une des revendications 1 à 6, caractérisé en ce que l'augmentation de la pression et le chargement de l'adsorbeur dans la phase d'adsorption ont ensemble une durée de l'ordre d'environ la moitié jusqu'au double de la durée de la phase de rinçage ou de la phase de désorption.

8. Procédé suivant l'une des revendications 1 à 7, caractérisé en ce que la désorption de l'adsorbeur est réalisée en deux stades, le premier stade consistant à extraire de l'adsorbeur, par une première pompe à vide, à une pression qui diminue de la pression de gaz de rinçage à une pression intermédiaire, un mélange gazeux destiné au rinçage de l'un des autres adsorbeurs, et le deuxième stade immédiatement subséquent à aspirer par une deuxième pompe à vide, à une pression décroissant de la pression intermédiaire jusqu'à la pression finale de l'évacuation, le gaz de production.

9. Procédé suivant la revendication 8, caractérisé en ce que la pression intermédiaire est supérieure à la pression finale de 0,1 à 0,95 et de préférence de 0,4 à 0,8 fois la différence entre la pression de gaz de rinçage et la pression finale de l'évacuation et cette dernière se situe entre 0,01 et 0,6 et de préférence entre 0,03 et 0,2 bar.

10. Procédé suivant l'une des revendications 1 à 9, caractérisé en ce que le rinçage de l'adsorbeur (la phase de rinçage) est réalisé à une pression inférieure à la pression de chargement (phase d'adsorption).

11. Procédé suivant la revendication 10, caractérisé en ce que, après un chargement à une pression supérieure à la pression atmosphérique et de préférence supérieure à 3 bars, le produit gazeux extrait pendant l'abaissement de la pression à la pression de gaz de rinçage est éliminé (gaz résiduaire) aussi longtemps que les proportions en volume du ou des composants plus fortement adsorbés (à séparer) sont inférieures à celles du mélange gazeux brut (mélange de départ).

12. Procédé suivant la revendication 10, caractérisé en ce que, après un chargement à une pression supérieure à la pression atmosphérique et de préférence supérieure à 3 bars, le produit gazeux extrait pendant l'abaissement de la pression (à la pression

**0 146 646**

de gaz de rinçage) est utilisé pour l'augmentation de la pression dans l'un des autres adsorbeurs.

13. Procédé suivant les revendications 10 à 12, caractérisé en ce que, après un chargement à une pression supérieure à la pression atmosphérique et de préférence supérieure à 3 bars, le produit gazeux soutiré pendant l'abaissement de la pression est amené à un compresseur pour le mélange gazeux brut à partir du moment où les proportions en volume du ou des composants plus fortement adsorbés (à séparer) sont devenues supérieures à celles du mélange gazeux brut.

14. Procédé suivant l'une des revendications 10 à 13, caractérisé en ce que la pression de gaz de rinçage est atteinte au moment où la proportion en volume du ou des composants à séparer dépasse dans le mélange gazeux soutiré une valeur prédéterminée, qui se situe entre la proportion moyenne en volume dans les gaz de production et la proportion en volume dans le mélange gazeux brut.

FIG. 1

| Ventil Nr. | Phase 1 | Phase 2 | Phase 3 | Phase 4 |
|---|---|---|---|---|
| 5 | | | | |
| 9 | | | | |
| 1 3 | | | | |
| 1 7 | | | | |
| 2 1 | | | | |
| | | | | |
| 6 | | | | |
| 1 0 | | | | |
| 1 4 | | | | |
| 1 8 | | | | |
| 2 2 | | | | |
| | | | | |
| 7 | | | | |
| 1 1 | | | | |
| 1 5 | | | | |
| 1 9 | | | | |
| 2 3 | | | | |
| | | | | |
| 8 | | | | |
| 1 2 | | | | |
| 1 6 | | | | |
| 2 0 | | | | |
| 2 4 | | | | |

Druck

1

0

**FIG.2**

Zeit

FIG.3

# FIG. 4

| Ventil Nr. | Phase 1 | Phase 2 | Phase 3 | Phase 4 |
|---|---|---|---|---|
| 111 | | | | |
| 112 | | | | |
| 113 | | | | |
| 115 | | | | |
| 116 | | | | |
| 117 | | | | |
| 121 | | | | |
| 122 | | | | |
| 123 | | | | |
| 125 | | | | |
| 126 | | | | |
| 127 | | | | |
| 131 | | | | |
| 132 | | | | |
| 133 | | | | |
| 135 | | | | |
| 136 | | | | |
| 137 | | | | |
| 141 | | | | |
| 142 | | | | |
| 143 | | | | |
| 145 | | | | |
| 146 | | | | |
| 147 | | | | |

Druck

1

0

Zeit

FIG. 5

0 146 646

# FIG. 6

| Ventil Nr. | Phase 1 | Phase 2 | Phase 3 | Phase 4 |
|---|---|---|---|---|
| 211 | ✕ | | ✕ | |
| 212 | | | ✕ | |
| 213 | | ✕ | | |
| 214 | | | | ✕ |
| 215 | ✕ | ✕ | | |
| 216 | | ✕ | | |
| | | | | |
| 221 | | ✕ | | ✕ |
| 222 | | | | ✕ |
| 223 | | | ✕ | |
| 224 | ✕ | | | |
| 225 | | ✕ | ✕ | |
| 226 | | | ✕ | |
| | | | | |
| 231 | | ✕ | ✕ | |
| 232 | ✕ | | | ✕ |
| 233 | | | | ✕ |
| 234 | | ✕ | | |
| 235 | | | ✕ | ✕ |
| 236 | | | | ✕ |
| | | | | |
| 241 | | | | ✕ |
| 242 | | ✕ | | |
| 243 | ✕ | | | |
| 244 | | | ✕ | |
| 245 | ✕ | | | ✕ |
| 246 | | ✕ | | |

Druck
1
0
Zeit

FIG. 7

# FIG. 8